# EUROPEAN PATENT APPLICATION

(11) **EP 4 623 850 A1**
(43) Date of publication of application: **01.10.2025**
(21) Application number: 23893565.4
(22) Date of filing: 31.10.2023
(51) Int. Cl.: A61B 34/30

(54) **ROBOTIC ARM FOR SURGICAL ROBOT AND SURGICAL ROBOT**

(30) Priority: 24.11.2022 CN 202211483980
(71) Applicant: Ronovo (Shanghai) Medical Science and Technology Ltd., Shanghai 201102 (CN)
(72) Inventor: MAO, Ying, Shanghai 201102 (CN); SUN, Pei, Shanghai 201102 (CN); JIN, Xin, Shanghai 201102 (CN); LI, Gengyi, Shanghai 201102 (CN); ZHENG, Qi, Shanghai 201102 (CN); AN, Shuyang, Shanghai 201102 (CN)
(74) Representative: Cabinet Beaumont
(86) International application number: PCT/CN2023/128111
(87) International publication number: WO 2024/109474

(57) **Abstract**

Provided are a robotic arm for a surgical robot and a surgical robot. The robotic arm includes an adjustment arm, a tool arm, and a surgical tool linear guide (60). The adjustment arm includes a first adjustment rotary joint (11), an adjustment rod, and at least two second adjustment rotary joints. The rotation axis of the first adjustment rotary joint (11) extends vertically. One of the at least two second adjustment rotary joints is disposed on the first adjustment rotary joint (11). Two adjacent second adjustment rotary joints are connected by the adjustment rod. The rotation axis of a second adjustment rotary joint extends horizontally. The tool arm includes a first tool rotary joint (15) and a tool arm body. The first tool rotary joint (15) is connected to the adjustment arm. The rotation axis of the first tool rotary joint (15) extends horizontally. The tool arm body is connected to the surgical tool linear guide (60).

## Description

This application claims priority to Chinese Patent Application No. 202211483980.6 filed Nov. 24, 2022, the disclosure of which is incorporated herein by reference in its entirety.

### TECHNICAL FIELD

The present application relates to the technical field of medical devices, for example, to a robotic arm for a surgical robot and a surgical robot.

### BACKGROUND

Laparoscopic robots in the related art typically include a robotic arm mounted on a cart. The robotic arm may be divided into an adjustment arm and a tool arm. Once the cart is fixed in position, the position of each joint of the adjustment arm may be adjusted to position the end of the tool arm at an appropriate location in space to perform surgery. Generally, the adjustment arm of a surgical robot adopts a SCARA (selective compliance assembly robot arm) configuration, which is simple in design but has the following issues.
1. The arm rods of the robotic arm need to occupy significant lateral space, taking up valuable bedside space, encroaching on the standing positions of assistants and nurses, and increasing the likelihood of collisions with the robotic arm.
2. The learning curve for null space adjustment is steep, making it difficult for assistants and nurses to intuitively adjust the posture of the robotic arm to ensure sufficient motion space for the robot to complete surgery.
3. Null space adjustment is challenging, requiring assistants to manually adjust numerous joints while ensuring that the end of the instrument and the remote center of motion remain stationary.

### SUMMARY

The present application provides a robotic arm for a surgical robot and a surgical robot, which can reduce the bedside space occupied by the robotic arm and facilitate null space adjustment.

An embodiment provides a robotic arm for a surgical robot. The robotic arm includes an adjustment arm, a tool arm, and a surgical tool linear guide.

The adjustment arm includes a first adjustment rotary joint, an adjustment rod, and at least two second adjustment rotary joints. The rotation axis of the first adjustment rotary joint extends vertically. One second adjustment rotary joint is disposed on the first adjustment rotary joint. Two adjacent second adjustment rotary joints are connected by the adjustment rod. The rotation axis of a second adjustment rotary joint extends horizontally.

The tool arm includes a first tool rotary joint and a tool arm body connected in sequence. The first tool rotary joint is connected to the adjustment arm.

The surgical tool linear guide is connected to an end of the tool arm body and is configured to connect to a surgical tool. The tool arm cooperates with the surgical tool linear guide to achieve posture adjustment of the surgical tool relative to a remote center of motion.

An embodiment provides a surgical robot. The surgical robot includes a surgical tool and the preceding robotic arm for a surgical robot. The surgical tool is mounted at an end of the robotic arm. The robotic arm is configured to adjust a position and a posture of the surgical tool.

### BRIEF DESCRIPTION OF DRAWINGS

FIG. 1 is a first view illustrating the structure of a surgical robot according to embodiment one of the present application.
FIG. 2 is a second view illustrating the structure of the surgical robot according to embodiment one of the present application.
FIG. 3 is a view illustrating the structure of a surgical robot according to embodiment one of the present application when a first tool rod is parallel to the ground.
FIG. 4 is a view illustrating the structure of a surgical robot according to embodiment one of the present application when a first tool rod forms a 15° angle with the ground.
FIG. 5 is a view illustrating the structure of a surgical robot according to embodiment one of the present application when a first tool rod forms a -15° angle with the ground.
FIG. 6 is a view illustrating part of the structure of a surgical robot according to embodiment one of the present application.
FIG. 7 is a view illustrating part of the structure of a surgical robot according to embodiment two of the present application.
FIG. 8 is a view of a light indication mechanism of a surgical robot according to embodiment two of the present application.
FIG. 9 is a view of a surgical robot according to embodiment one of the present application.
FIG. 10 is a view illustrating the structure of a feed drive according to embodiment one of the present application.
FIG. 11 is a view of a tool arm joint and a drive assembly according to embodiment one of the present application.

### Reference list

- 11: first adjustment rotary joint
- 12: third adjustment rotary joint
- 13: fourth adjustment rotary joint
- 131: first display component
- 14: fifth adjustment rotary joint
- 15: first tool rotary joint
- 16: third tool rotary joint
- 17: fourth tool rotary joint
- 18: fifth tool rotary joint
- 19: second tool rotary joint
- 21: first arm rod
- 22: second arm rod
- 23: first tool rod
- 24: second tool rod
- 25: third tool rod
- 30: surgical tool
- 32: instrument driver
- 33: surgical instrument
- 41: null space adjustment button
- 42: angle indicator light group
- 51: second display component
- 52: third display component
- 60: surgical tool linear guide
- 70: cart
- 80: encoding block
- 90: feed drive
- 100: tool arm joint
- 110: drive assembly

### DETAILED DESCRIPTION

In the description of the present application, unless otherwise expressly specified and limited, a term "connected to each other", "connected", or "secured" is to be construed in a broad sense, for example, as securely connected, detachably connected, or integrated; mechanically connected or electrically connected; directly connected to each other or indirectly connected to each other via an intermediary; or internally connected between two components or interaction relations between two components. For those of ordinary skill in the art, specific meanings of the preceding terms in the present application may be construed according to specific situations.

In the present application, unless otherwise expressly specified and limited, when a first feature is described as "above" or "below" a second feature, the first feature and the second feature may be in direct contact or be in contact via another feature between the two features instead of being in direct contact. Moreover, when the first feature is "on", "above", or "over" the second feature, the first feature is right on, above, or over the second feature, or the first feature is obliquely on, above, or over the second feature, or the first feature is simply at a higher level than the second feature. When the first feature is "under", "below", or "underneath" the second feature, the first feature is right under, below, or underneath the second feature, or the first feature is obliquely under, below, or underneath the second feature, or the first feature is simply at a lower level than the second feature.

In the description of the embodiments, orientations or position relations indicated by terms such as "above", "below", and "right" are based on orientations or position relations shown in the drawings. These orientations or position relations are intended only to facilitate and simplify the operation and not to indicate or imply that a device or element referred to must have such specific orientations or must be configured or operated in such specific orientations. Thus, these orientations or position relations are not to be construed as limiting the present application. In addition, terms "first" and "second" are used only to distinguish between descriptions and have no special meaning.

### Embodiment one

This embodiment provides a surgical robot. The surgical robot includes a surgical tool 30 and a robotic arm for a surgical robot. The surgical tool 30 is mounted at an end of the robotic arm. The robotic arm is configured to adjust a position and a posture of the surgical tool 30. The surgical tool 30 serves as an operating end, acting on a lesion site of a patient.

The surgical robot provided in the present application adopts the preceding robotic arm, featuring a flexible structure and occupying a small space.

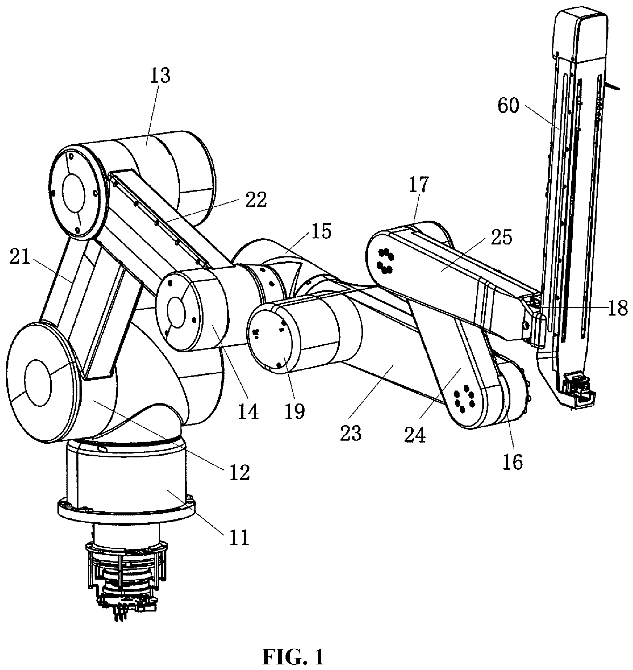

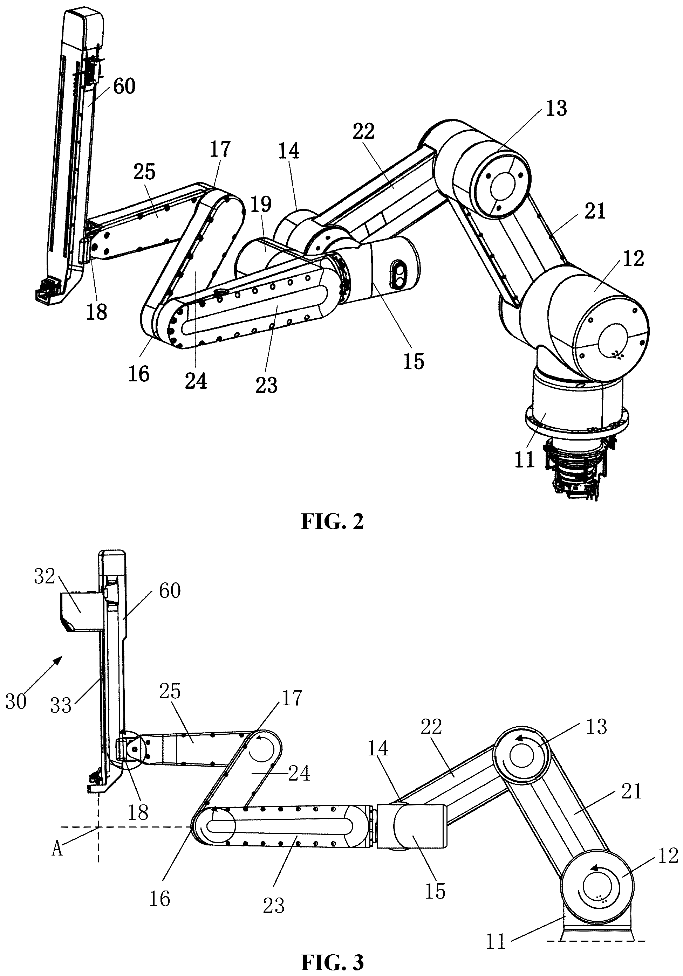

As shown in FIGS. 1 and 2, the robotic arm for a surgical robot includes an adjustment arm, a tool arm, and a surgical tool linear guide 60. The tool arm is disposed at an end of the adjustment arm. The surgical tool linear guide 60 is disposed at an end of the tool arm. The adjustment arm is configured to adjust a position of the tool arm in space. The tool arm is configured to adjust a posture of the surgical tool 30 to achieve the surgical tool 30 to perform surgery at an appropriate position and with an appropriate posture.

In an embodiment, the adjustment arm includes a first adjustment rotary joint 11 and at least two second adjustment rotary joints arranged in sequence, and two adjacent second adjustment rotary joints are connected by an adjustment rod. In this embodiment, three second adjustment rotary joints are provided, and two adjustment rods are provided accordingly.

For ease of description, the three second adjustment rotary joints are designated as a third adjustment rotary joint 12, a fourth adjustment rotary joint 13, and a fifth adjustment rotary joint 14, and the two adjustment rods are designated as a first arm rod 21 and a second arm rod 22. The rotation axis of the first adjustment rotary joint 11 extends vertically, and rotation axes of the third adjustment rotary joint 12, the fourth adjustment rotary joint 13, and the fifth adjustment rotary joint 14 are parallel to each other and extend horizontally.

The tool arm includes a first tool rotary joint 15 and a tool arm body connected in sequence. The tool arm body is configured to mount the surgical tool linear guide 60 and is capable of forming a parallelogram linkage structure with the surgical tool linear guide 60 to achieve posture adjustment of the surgical tool 30 relative to a remote center of motion.

In the robotic arm for a surgical robot provided in the present application, the rotation axis of the first adjustment rotary joint 11 extends vertically so that the placement angle of the robotic arm beside the surgical bed can be flexibly adjusted as needed; the rotation axes of the second adjustment rotary joints extend horizontally, ensuring that the robotic arm primarily occupies longitudinal space beside the surgical bed regardless of adjustments of the robotic arm. The first tool rotary joint 15 provides an axial rotation degree of freedom for the tool arm body. The tool arm body cooperates with the surgical tool linear guide 60 to achieve posture adjustment of the surgical tool 30 at the remote center of motion. In this manner, the robotic arm can move flexibly and occupies small lateral space beside the surgical bed.

In an embodiment, the tool arm body includes a first tool rod 23, a third tool rotary joint 16, a second tool rod 24, a fourth tool rotary joint 17, a third tool rod 25, and a fifth tool rotary joint 18, which are connected in sequence. The first tool rotary joint 15 is connected to the fifth adjustment rotary joint 14. Rotation axes of a second tool rotary joint 19, the third tool rotary joint 16, the fourth tool rotary joint 17, and the fifth tool rotary joint 18 extend horizontally. The second tool rotary joint 19, the third tool rotary joint 16, the fourth tool rotary joint 17, and the fifth tool rotary joint 18 are parallel to each other. The rotation axes of the second tool rotary joint 19, the third tool rotary joint 16, the fourth tool rotary joint 17, and the fifth tool rotary joint 18 may be set to be parallel to or form a preset angle with the rotation axis of the third adjustment rotary joint 12. The third tool rotary joint 16, the second tool rod 24, the fourth tool rotary joint 17, the third tool rod 25, and the fifth tool rotary joint 18 are capable of forming a parallelogram linkage structure with the surgical tool linear guide 60.

In this embodiment, the adjustment arm has four independent rotary joints, which can adjust the position of the surgical tool 30 disposed at the end of the tool arm in three-dimensional space. The rotation axes of the third adjustment rotary joint 12, the fourth adjustment rotary joint 13, and the fifth adjustment rotary joint 14 extend horizontally. Thus, the first arm rod 21 and the second arm rod 22 rotate in a vertical plane, occupying a small space and conserving bedside space.

The adjustment arm provides a total of four degrees of freedom, including one redundant degree of freedom. In this manner, degradation of one or more degrees of freedom is prevented from affecting the flexibility of the robotic arm, collisions between robotic arms can be avoided, additional workspace is obtained, and the driving torque of each joint is optimized.

In an embodiment, considering that the motion of the third adjustment rotary joint 12 and the fourth adjustment rotary joint 13 can expand the motion range of the tool arm, the redundant degree of freedom is preferentially assigned to the fifth adjustment rotary joint 14.

During minimally invasive surgery, the surgical tool 30 needs to perform fixed-point motion (that is, remote center motion) constrained by the body surface incision of the patient. The location of the body surface incision of the patient serves as the remote center of motion.

In this embodiment, the first tool rod 23, the third tool rotary joint 16, the second tool rod 24, the fourth tool rotary joint 17, the third tool rod 25, and the fifth tool rotary joint 18 in the tool arm can form a parallelogram linkage structure with the surgical tool linear guide 60, enabling the surgical tool 30 mounted on the surgical tool linear guide 60 to perform fixed-point motion. The first tool rotary joint 15 provides an axial rotation degree of freedom for the surgical tool 30.

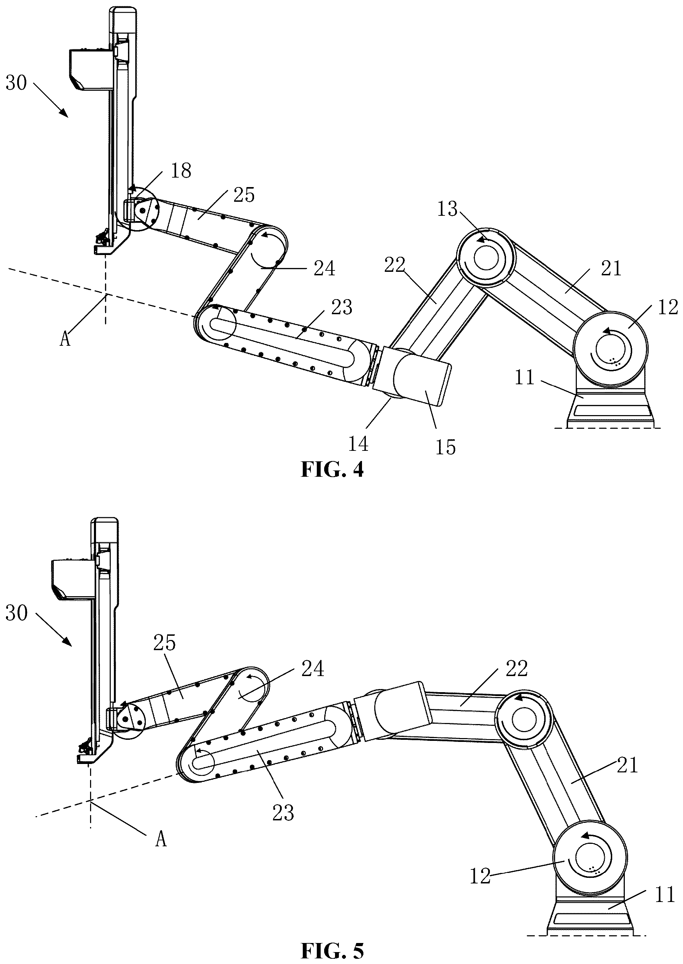

In an embodiment, the first tool rod 23 may be regarded as a frame of the parallelogram linkage structure. By the adjustment of the posture of the adjustment arm, the extension direction of the first tool rod 23 can be modified, and thus the angle between the first tool rod 23 and the ground can vary as needed. As shown in FIG. 3, the first tool rod 23 is arranged parallel to the ground. In FIG. 4, the first tool rod 23 forms a 15° angle with the ground. In FIG. 5, the first tool rod 23 forms a -15° angle with the ground.

In summary, the adjustment arm in this embodiment provides three spatial translational degrees of freedom and one redundant degree of freedom for the tool arm, and the tool arm provides two rotational degrees of freedom and one translational degree of freedom for the surgical tool 30, so as to enhance the flexibility of the robotic arm during use.

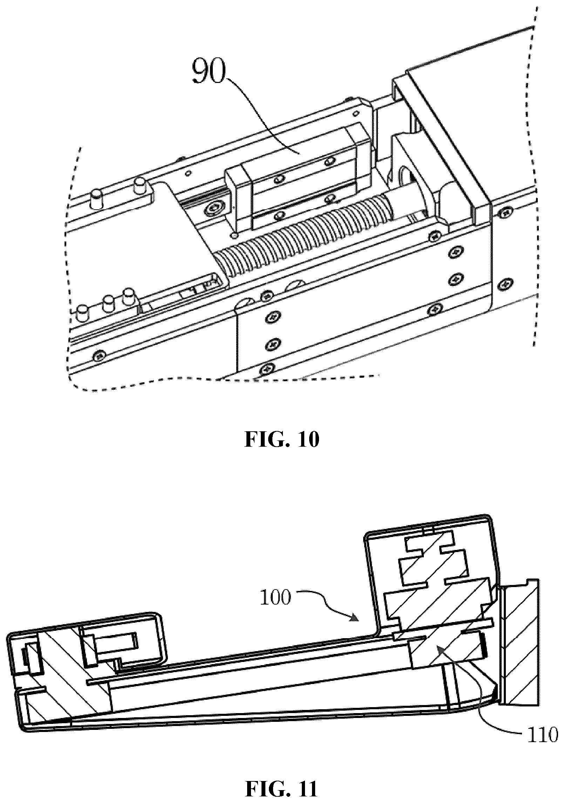

In some embodiments, the tool arm does not need to form a parallelogram linkage structure with the surgical tool linear guide 60. Instead, a drive assembly 110 is disposed at each joint of the tool arm to simulate a parallelogram linkage structure to control the surgical tool 30 to perform fixed-point motion via electronic control. In an embodiment, as shown in FIG. 11, the tool arm body includes multiple tool arm joints 100 and drive assemblies 110 configured to drive motion of the tool arm joints 100. Each tool arm joint 100 is provided with a respective drive assembly 110 to control the surgical tool 30 to perform fixed-point motion.

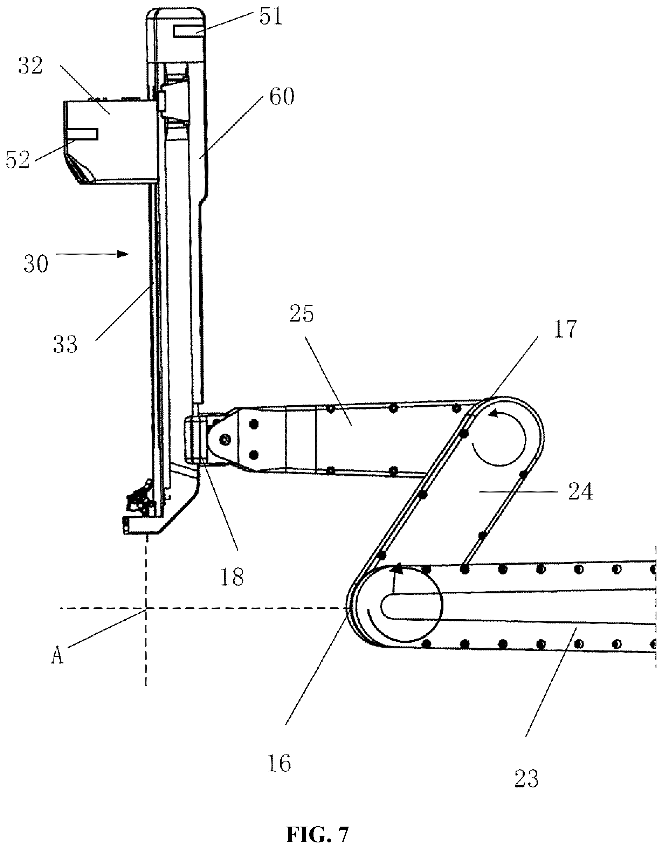

In an embodiment, as shown in FIG. 7 and FIG. 10, the surgical tool 30 includes a feed drive 90, an instrument driver 32, and a surgical instrument 33. The surgical tool linear guide 60 is disposed on the fifth tool rotary joint 18. The feed drive 90 is disposed on the surgical tool linear guide 60. The instrument driver 32 is connected to the feed drive 90. The feed drive 90 is configured to drive the instrument driver 32 to move along a length direction of the surgical tool linear guide 60. The surgical instrument 33 is connected to the instrument driver 32. The instrument driver 32 is configured to drive the surgical instrument 33 to rotate around the axis of the surgical instrument 33. The feed drive 90 is configured to adjust an insertion depth of the surgical instrument 33 without providing a degree of freedom for the remote center of motion.

In this embodiment, the surgical robot has six degrees of freedom, including three translational degrees of freedom, two rotational degrees of freedom, and one redundant degree of freedom, thereby enabling a more flexible spatial arrangement. The first arm rod 21, the second arm rod 22, the first tool rod 23, the second tool rod 24, and the third tool rod 25 can move in a vertical plane. Therefore, they can be folded and stored beside the surgical bed with a small footprint.

To further reduce the space occupied by the robotic arm beside the surgical bed, the length of the first arm rod 21 is equal to or greater than the length of the second arm rod 22 so that the second arm rod 22 can fold to a position substantially overlapping the first arm rod 21 when the third adjustment rotary joint 12 drives the second arm rod 22 to rotate, thereby facilitating folding during storage. Additionally, the shorter length of the second arm rod 22 ensures an appropriate motion range of the second arm rod 22, thereby avoiding interference with the surgical bed due to an excessively long second arm rod 22 and preventing a too-short second arm rod 22 from limiting the adjustment range.

To enable the robotic arm to maintain its current posture after adjustment, each joint in the adjustment arm and the tool arm is provided with a corresponding brake. When the brake is locked, the corresponding joint is fixed and cannot be adjusted. When the brake is released, the corresponding joint may be manually adjusted by a user or automatically adjusted to modify the posture and position of the robotic arm.

During surgery, the tool arm needs to drive the surgical tool 30 to operate, so the first tool rotary joint 15, the second tool rotary joint 19, the third tool rotary joint 16, the fourth tool rotary joint 17, and the fifth tool rotary joint 18 are active joints.

The adjustment arm may be adjusted in posture before surgery to move to a designated position, either by manual dragging or computer-controlled movement. Thus, in the adjustment arm, the first adjustment rotary joint 11, the third adjustment rotary joint 12, the fourth adjustment rotary joint 13, and the fifth adjustment rotary joint 14 may be passive joints or active joints.

It should be noted that the brake is a common structure at a passive joint in the robotic arm and is a mature technology in the field. The brake in this embodiment may adopt any brake structure and operating principle from the related art, and no specific description is provided herein.

In some embodiments, the first adjustment rotary joint 11, the third adjustment rotary joint 12, the fourth adjustment rotary joint 13, the fifth adjustment rotary joint 14, the first tool rotary joint 15, the third tool rotary joint 16, the fourth tool rotary joint 17, and the fifth tool rotary joint 18 may be provided with drive assemblies to drive rotation of the corresponding joints. In an embodiment, the drive assembly includes a motor and a reducer connected to the motor. The reducer is connected to the corresponding joint to drive the joint to rotate after the speed and torque of the motor are adjusted via the reducer.

In an embodiment, the first arm rod 21 is disposed on a radial side of the first adjustment rotary joint 11, the second arm rod 22 is disposed on one side of the first arm rod 21 facing away from the first adjustment rotary joint 11, and the tool arm is disposed on one side of the second arm rod 22 facing the first adjustment rotary joint 11. This arrangement can minimize the distance between the axis of the first tool rotary joint 15 and the axis of the first adjustment rotary joint 11, reduce the radial dimension of the robotic arm along the first adjustment rotary joint 11, and ensure that the robotic arm, regardless of adjustments, primarily occupies longitudinal space beside the surgical bed for ease of use.

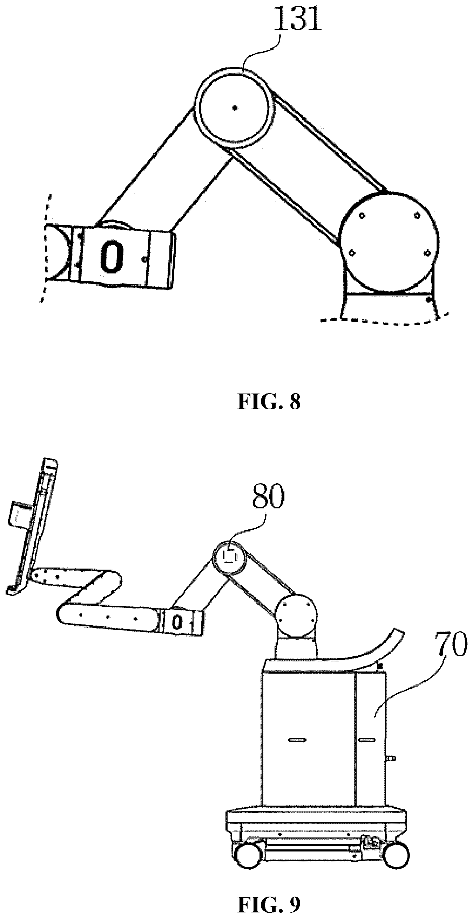

In an embodiment, as shown in FIG. 9, the robotic arm for a surgical robot also includes a cart 70, and the first adjustment rotary joint 11 is disposed on the cart 70. The adjustment arm is mounted on the cart 70 so that the position of the robotic arm can be adjusted by moving the cart 70, thus facilitating positioning beside the surgical bed. In an embodiment, the cart 70 is provided with wheels at its bottom, making movement of the cart 70 more convenient and labor-saving.

In an embodiment, the height of the cart 70 is not lower than the height of a surgical bed carrying a patient. In this manner, it is ensured that the motion range of the first arm rod 21 remains above the surgical bed to avoid interference between the robotic arm and the surgical bed.

In an embodiment, the height of the cart 70 is adjustable to accommodate surgical beds of different heights, ensuring that the motion range of the first arm rod 21 remains above the surgical bed to prevent interference.

In an embodiment, a gravity compensation device, such as a mechanical gravity compensation device, may also be disposed in the robotic arm to counteract the influence of the weight of the robotic arm on its posture and position adjustments. It should be noted that the gravity compensation device is a common structure in the field of robotic arms and is a mature technology. The gravity compensation device in this embodiment may adopt the structure and working principle of any gravity compensation device from the related art, and no specific description is provided herein.

In an embodiment, the gravity compensation device may be disposed in the first tool rod 23.

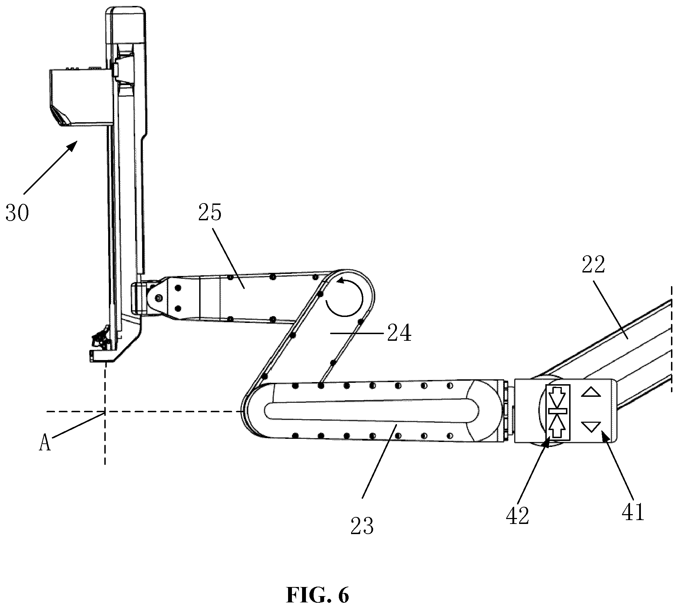

In an embodiment, as shown in FIG. 6, the first tool rotary joint 15 may also be provided with a null space adjustment button 41 and an angle indicator light group 42.

When the end of the robotic arm remains stationary (that is, the surgical tool 30 is immobile), the configuration in which other joints of the robotic arm can rotate constitutes the null space. The null space adjustment button includes an upper adjustment button and a lower adjustment button. The upper adjustment button is configured to drive the first tool rod 23 to move upward. The lower adjustment button is configured to drive the first tool rod 23 to move downward. Null space adjustment via the null space adjustment button 41 allows the first tool rod 23 to be lowered as close as possible to the patient and the surgical bed, freeing up space above the adjustment arm and the tool arm to avoid collisions between robotic arms during surgery. Additionally, adjusting the robotic arm in the vertical direction facilitates observation by doctors to confirm proper adjustment.

The angle indicator light group 42 includes an upper adjustment indicator light, a lower adjustment indicator light, and an extension direction indicator light and is configured to cooperate with the null space adjustment button to display the current state of the first tool rod 23.

### Embodiment two

This embodiment provides a surgical robot that is further improved based on embodiment one. In an embodiment, the surgical robot also includes a light indication mechanism. The light indication mechanism is configured to display light in coordination with the state of the robotic arm and the state of the surgical tool 30 to prevent medical staff from applying excessive force to the robotic arm, where excessive force leads to inaccurate posture of the robotic arm. The light indication mechanism may provide indications through different colors, brightness variations, or flashing patterns.

In an embodiment, as shown in FIG. 8, the light indication mechanism includes first display components 131, and at least one second adjustment rotary joint is provided with the first display components 131 at two ends in an axial direction. The first display component 131 may be configured to indicate an operating state and/or a motion state of the adjustment arm.

In this embodiment, the first display components 131 are disposed on two axial end faces of the fourth adjustment rotary joint 13. In commonly used postures of the robotic arm during surgery, the fourth adjustment rotary joint 13 is at a relatively high position in the entire robotic arm. Positioning the first display components 131 on the fourth adjustment rotary joint 13 ensures that medical staff can observe the light color, brightness, and flashing status of the first display components 131 from multiple directions and distances.

In an embodiment, the first display component 131 includes a display screen or multiple light-emitting diodes (LEDs) arranged in an array. The first display component 131 may directly display information via the display screen or display text or image information through combinations of lit and unlit LEDs.

To facilitate observation by medical staff, the up and down direction of the information displayed by the first display component 131 is always in the vertical direction. In an embodiment, the surgical robot may calculate the angle of the fourth adjustment rotary joint 13 relative to the ground based on posture information of the robotic arm, thereby adjusting the angle of information displayed on the first display component 131 on the fourth adjustment rotary joint 13 based on the angle. Thus, it is ensured that the up and down direction of the information displayed on the first display component 131 is always in the vertical direction.

For ease of description, it is defined that the surgical tool 30 is located at a front side of the tool arm. As shown in FIG. 7, the light indication mechanism also includes a second display component 51. The second display component 51 is disposed at the top of the surgical tool linear guide 60 and extends along the circumference of the surgical tool linear guide 60 to at least two side surfaces of the top of the surgical tool linear guide 60. The second display component 51 is configured to indicate an operating state and/or a motion state of the tool arm, a successful docking state of the tool arm with a trocar, and a master-slave teleoperation state. In an embodiment, the second display component 51 is disposed at the top of the surgical tool linear guide 60 and is positioned at a relatively high location in commonly used robotic arm postures during surgery. Moreover, the second display component 51 surrounds the left, right, and rear sides of the surgical tool linear guide 60, ensuring that medical staff can observe the light color and brightness status of the second display component 51 from most directions and distances. In an embodiment, the second display component 51 may provide indications through light colors and flashing patterns.

In an embodiment, the light indication mechanism also includes a third display component 52. The third display component 52 is disposed at the top of the surgical tool 30 and extends along the circumference of the surgical tool 30 to at least two side surfaces of the top of the surgical tool 30. The third display component 52 is configured to indicate an installation state and/or a motion state of the surgical tool. In an embodiment, the third display component 52 is disposed at the top of the instrument driver 32 and extends from the left and right sides of the instrument driver 32 to a front side. The preceding arrangement ensures that medical staff can observe the light color and brightness status of the third display component 52 from most directions and distances. In an embodiment, the third display component 52 may provide indications through light colors and flashing patterns.

In this embodiment, the cooperation of the first display components 131, the second display component 51, and the third display component 52 achieves the following: First, the states of the tool arm and the adjustment arm can be observed intuitively, reducing errors. Second, when the tool arm and adjustment arm are in motion, the light indication mechanism mounted on the robotic arm remains unobstructed. Third, the light indication mechanism can display multiple states using a limited number of indicator lights.

When multiple surgical robots need to be simultaneously integrated into a system for operation, each surgical robot may be numbered as needed to facilitate flexible arrangement and combined use. Each surgical robot may display its number via the first display component 131.

In some embodiments, the robotic arm is provided with an encoding block 80 that may cover an outer peripheral surface of a joint of the robotic arm. The encoding block 80 can rotate relative to the robotic arm j oint, and the rotation axis of the encoding block 80 is parallel to the rotation axis of the corresponding joint. The center of gravity of the encoding block 80 is eccentrically disposed relative to the rotation center of the encoding block 80. This arrangement ensures that, regardless of the posture of the adjustment arm, the encoding block 80 can rotate relative to the robotic arm joint under its own weight so that the encoding on the encoding block 80 is always in a horizontal state for easy viewing. Additionally, the encoding block 80 is provided with an encoding that clearly distinguishes multiple robotic arms during surgery, facilitating identification by doctors.

In an embodiment, the tool arm is provided with two adjustment arm clutch buttons. An adjustment arm clutch button is disposed at the bottom of each of the first tool rod 23 and the fifth tool rotary joint 18. Providing two adjustment arm clutch buttons ensures that assistants can perform the adjustment arm clutch operation from either the left or right side of the robotic arm. Positioning these function buttons at an end of the tool arm farther from the adjustment arm increases the lever arm of the adjustment arm, thus reducing the effort required for the adjustment arm clutch operation. The position of the adjustment arm clutch button on the first tool rod 23 allows the button to be more easily activated while the first tool rod 23 is held in a more comfortable and secure manner, thereby facilitating the adjustment arm clutch operation. Similarly, the position of the adjustment arm clutch button located at the bottom of the fifth tool rotary joint 18 allows the adjustment arm clutch function to be conveniently activated while the fifth tool rotary joint 18 is held in a more comfortable and secure manner, thereby enabling dragging to begin.

## Claims

1. A robotic arm for a surgical robot, comprising:
an adjustment arm, wherein the adjustment arm comprises a first adjustment rotary joint (11), an adjustment rod, and at least two second adjustment rotary joints, a rotation axis of the first adjustment rotary joint (11) extends vertically, one second adjustment rotary joint of the at least two second adjustment rotary joints is disposed on the first adjustment rotary joint (11), two adjacent second adjustment rotary joints are connected by the adjustment rod, and a rotation axis of the at least two second adjustment rotary joints extends horizontally;
a tool arm, wherein the tool arm comprises a first tool rotary joint (15) and a tool arm body connected in sequence, and the first tool rotary joint (15) is connected to the adjustment arm; and
a surgical tool linear guide (60), wherein the surgical tool linear guide (60) is connected to an end of the tool arm body and is configured to connect to a surgical tool (30), and the tool arm cooperates with the surgical tool linear guide (60) to achieve posture adjustment of the surgical tool (30) relative to a remote center of motion.

2. The robotic arm for the surgical robot according to claim 1, wherein the tool arm body comprises a second tool rotary joint (19), a first tool rod (23), a third tool rotary joint (16), a second tool rod (24), a fourth tool rotary joint (17), a third tool rod (25), and a fifth tool rotary joint (18), which are connected in sequence;
wherein a rotation axis of the second tool rotary joint (19), a rotation axis of the third tool rotary joint (16), a rotation axis of the fourth tool rotary joint (17), and a rotation axis of the fifth tool rotary joint (18) are parallel to each other and extend horizontally; the fifth tool rotary joint (18) is connected to the surgical tool linear guide (60), and the third tool rotary joint (16), the second tool rod (24), the fourth tool rotary joint (17), the third tool rod (25), and the fifth tool rotary joint (18) are configured to form a parallelogram linkage structure with the surgical tool linear guide (60).

3. The robotic arm for the surgical robot according to claim 1, wherein the tool arm body comprises a plurality of tool arm joints (100) and drive assemblies (110) configured to drive motion of the plurality of tool arm joints (100), each tool arm j oint (100) of the plurality of tool arm joints (100) is provided with a respective one of the drive assemblies (110) to enable the surgical tool (30) to perform motion relative to the remote center of motion.

4. The robotic arm for the surgical robot according to any one of claims 1 to 3, further comprising a cart (70), wherein the first adjustment rotary joint (11) is disposed on the cart (70).

5. The robotic arm for the surgical robot according to claim 4, wherein a height of the cart (70) is not lower than a height of a surgical bed carrying a patient.

6. The robotic arm for the surgical robot according to claim 4, wherein a height of the cart (70) is adjustable.

7. The robotic arm for the surgical robot according to any one of claims 1 to 3, wherein the adjustment arm comprises three second adjustment rotary joints, and the three second adjustment rotary joints are sequentially connected by two adjustment rods.

8. The robotic arm for the surgical robot according to claim 7, wherein in a direction away from the first adjustment rotary joint (11), a redundant joint is a second adjustment rotary joint located at an end of the direction.

9. The robotic arm for the surgical robot according to claim 7, wherein a length of an adjustment rod proximal to the first adjustment rotary joint (11) is equal to or greater than a length of an adjustment rod distal to the first adjustment rotary joint (11).

10. The robotic arm for the surgical robot according to claim 7, wherein the two adjustment rods are a first arm rod (21) and a second arm rod (22), respectively, the first arm rod (21) is connected to the one second adjustment rotary joint disposed on the first adjustment rotary joint (11), the first arm rod (21) is disposed on a radial side of the first adjustment rotary joint (11), the second arm rod (22) is disposed on one side of the first arm rod (21) facing away from the first adjustment rotary joint (11), and the tool arm is disposed on one side of the second arm rod (22) facing the first adjustment rotary joint (11).

11. The robotic arm for the surgical robot according to any one of claims 1 to 3, wherein the first adjustment rotary joint (11) and the at least two second adjustment rotary joints are passive joints.

12. The robotic arm for the surgical robot according to any one of claims 1 to 3, further comprising an encoding block (80), wherein the encoding block (80) is disposed on an outer peripheral surface of one second adjustment rotary joint of the at least two second adjustment rotary joints and is configured to rotate relative to the one second adjustment rotary joint, a rotation axis of the encoding block (80) is parallel to a rotation axis of the one second adjustment rotary joint on which the encoding block (80) is disposed, the encoding block (80) is provided with an encoding, and a center of gravity of the encoding block (80) is eccentrically disposed relative to a rotation center of the encoding block (80).

13. A surgical robot, comprising a surgical tool (30) and the robotic arm for the surgical robot according to any one of claims 1 to 12, wherein the surgical tool (30) is mounted at an end of the robotic arm, and the robotic arm is configured to adjust a position and a posture of the surgical tool (30).

14. The surgical robot according to claim 13, wherein the surgical tool (30) comprises:
a feed drive (90) disposed on the surgical tool linear guide (60);
an instrument driver (32) connected to the feed drive (90), wherein the feed drive (90) is configured to drive the instrument driver (32) to move in a vertical direction; and
a surgical instrument (33) connected to the instrument driver (32), wherein the instrument driver (32) is configured to drive the surgical instrument (33) to rotate around an axis of the surgical instrument (33).

15. The surgical robot according to claim 13, further comprising a light indication mechanism, wherein the light indication mechanism is configured to display light in coordination with a state of the robotic arm and a state of the surgical tool (30).

16. The surgical robot according to claim 15, wherein the light indication mechanism comprises:
first display components (131), wherein at least one second adjustment rotary joint of the at least two second adjustment rotary joints is provided with the first display components (131) at two ends in an axial direction, and the first display components (131) are configured to indicate at least one of an operating state of the adjustment arm or a motion state of the adjustment arm.

17. The surgical robot according to claim 16, wherein three second adjustment rotary joints are provided, and the first display components (131) are disposed on a second adjustment rotary joint located in the middle.

18. The surgical robot according to claim 16, wherein each of the first display components (131) comprises a display screen or a plurality of light-emitting diodes, LEDs, arranged in an array, and the first display components (131) are configured to adjust an angle of displayed information based on a posture of the robotic arm to achieve horizontal display of the information.

19. The surgical robot according to claim 15, wherein the light indication mechanism comprises:
a second display component (51), wherein the surgical tool (30) is located at a front side of the tool arm, the second display component (51) extends from a left side and a right side of an upper end of the surgical tool (30) to a rear side of a top of the surgical tool (30), and the second display component (51) is configured to indicate at least one of an operating state of the tool arm or a motion state of the tool arm, a docking state of the tool arm with a trocar, and a master-slave teleoperation state.

20. The surgical robot according to claim 15, wherein the light indication mechanism comprises:
a third display component (52), wherein the third display component (52) extends from a left side and a right side of an upper end of the surgical tool (30) to a front side of a top of the surgical tool (30), and the third display component is configured to indicate at least one of an installation state of the surgical tool (30) or a motion state of the surgical tool (30).
